# EUROPÄISCHE PATENTSCHRIFT

(11) **EP 1 945 331 B1**
(45) Veröffentlichungstag und Bekanntmachung des Hinweises auf die Patenterteilung: **03.03.2010**
(21) Anmeldenummer: 06818354.0
(22) Anmeldetag: 03.11.2006
(51) Int. Cl.: B01D 53/86, A61L 9/014

(54) **VERFAHREN ZUR ELIMINIERUNG VON SCHWEFELWASSERSTOFF UND GERÜCHEN AUS ABWASSERSYSTEMEN**
PROCESS FOR ELIMINATING HYDROGEN SULPHIDE AND ODOURS FROM WASTEWATER SYSTEMS
PROCEDE D'ELIMINATION DU SULFURE D'HYDROGENE ET DES ODEURS D'UN RESEAU D'EGOUT

(30) Priorität: 09.11.2005 DE 102005053300
(43) Veröffentlichungstag der Anmeldung: 23.07.2008
(73) Patentinhaber: ROMOLD GmbH, 83395 Freilassing (DE)
(72) Erfinder: MÜLLER, Bernd, R., 17166 Teterow (DE)
(74) Vertreter: Thaler, Bernhard
(86) Internationale Anmeldenummer: PCT/EP2006/010544
(87) Internationale Veröffentlichungsnummer: WO 2007/054236

(56) Entgegenhaltungen:
- EP-A- 0 643 014
- EP-A1- 0 761 239
- US-A- 5 494 587
- US-A1- 2003 113 246
- K.-D. HENNING AND S. SCHÄFER: "Impregnated activated carbon for environmental protection" , [Online] 8 April 2005 (2005-04-08), Retrieved from the Internet: URL:http://web.archive.org/web/20050408034 843/http://www.activated-carbon.com/enviro .html> [retrieved on 2007-04-27]

## Beschreibung

Die Erfindung betrifft ein Verfahren zur Eliminierung von Schwefelwasserstoff und Gerüchen aus Abwassersystemen unter Verwendung von Aktivkohle.

Aktivkohle eignet sich sehr gut zur Abscheidung unpolarer Substanzen wie zum Beispiel Butan und Toluol. Hier können hohe Beladungskapazitäten von bis zu 50 Gew.-% bei sehr hoch aktivierten Aktivkohlen (Oberfläche etwa 1200 m²/g) erreicht werden. Temperaturen bei Raumtemperatur reichen aus, um diese physikalische Adsorption zu bewirken.

Herkömmliche Aktivkohlen können jedoch nur in geringem Maße polare Gase wie Schwefeldioxid, Formaldehyd, Ammoniak und Schwefelwasserstoff adsorbieren. Aus diesem Grunde werden Aktivkohlen zur Abscheidung von polaren Substanzen imprägniert. Die Imprägnierung ist ein Prozess, bei dem die Adsorbentien mit speziellen Reagenzien behandelt werden. Die auf die Poren der Aktivkohle gebrachten Reagenzien können dann mit den polaren Gasen reagieren und werden so an der imprägnierten inneren Oberfläche der Aktivkohle chemisorbiert. Die Reaktionszeit hierfür ist deutlich höher als bei der physikalischen Adsorption und beträgt einige Sekunden. Dieser Vorgang kann z. B. katalytische Reaktionen beinhalten.

Zur Entfernung von Schwefelwasserstoff aus Gasströmen kann die Aktivkohle mit basischen Verbindungen (z. B. mit KOH, NaOH etc.) imprägniert werden. Der Schwefelwasserstoff reagiert dann in Form einer Neutralisationsreaktion mit der auf der inneren Oberfläche aufgebrachten basischen Komponente. Das US Patent 5,024,682 offenbart den Einsatz von mit NaOH oder KOH imprägnierter Aktivkohle zur Entfernung von H₂S aus sauerstoffhaltigen Gasströmen.

Spezielle Imprägnierungen zur Entfernung von Schwefelwasserstoff aus Luft und Gasströmen sind bekannt. Bedeutend ist das Desorex-Verfahren (K. Storp, DECHEMA Monographie 64 (1970)). Bei diesem Verfahren wird die Aktivkohle aus einer heißen, wässrigen Lösung mit Kaliumcarbonat (K₂CO₃) zur katalytischen Oxidation von H₂S imprägniert. Hier wird Schwefelwasserstoff bei Einhaltung von Temperaturen um die 50 °C zu Sulfat in den Poren der Aktivkohle katalytisch oxidiert.

H₂S + K₂CO₃ (in Aktivkohle) + 2 O₂ → K₂SO₄ + CO₂ + H₂O

In EP 0761 239 A1 wird ein.katalytisches System zur Eliminierung von H2S und anderen schwefelhaltigen Verbindungen beschrieben, das sich von der vorliegenden Anmeldung wesentlich unterscheidet. In jener Anmeldunger handelt es sich nicht um dotierte Aktivkohlen, weil die katalytischen Komponenten (Mn02 und CuO) hauptsächlich auf der äußeren Oberfläche der Aktivkohle vorliegen. Die schwefelhaltigen Verbindungen reagieren zunächst mit den Metalloxiden, die sich auf der äußeren Oberfläche der Aktivkohle (auf dem Aktivkohlepulver) befinden, anschließend werden die Schwefelverbindungen von der Aktivkohle adsorbiert. Dies bedeutet, dass die Umsetzung der Schwefelverbindung (Methyltrisulfid, Methyltetrasulfid und Methyldisulfid) am MnO₂/CuO und die Adsorption (physikalische Adsorption) dieser Schwefelverbindungen räumlich getrennt ablaufen. Bei den erfindungsgemäß verwendeten dotierten Formaktivkohlen liegt hingegen das MnO₂ (Mangankomponente) und das K₂CO₃ hochdispers in den Poren (auch in den Mikroporen) der porösen Struktur der Aktivkohle vor (ohne die Mikroporen zu verengen) und damit in der inneren Oberfläche der Aktivkohle. Die physikalische Adsorption von H₂S und die Chemisorption von H₂S (Reaktion von H₂S mit K₂CO₃/MnO₂) laufen bei den dotierten, mikroporösen Aktivkohlen hauptsächlich in den Mikroporen ab. Beide Prozesse sind daher - entgegen der DI - nicht räumlich getrennt.

In US 2003/0113246 A1 werden nur imprägnierte Aktivkohlen beschrieben, die sich wesentlich von den erfindungsgemäß dotierten, mikroporösen Kohlen unterscheiden.

In US A-5 494 587 wird ein Verfahren beschrieben, in dem K₂CO₃ als Komponente zur Entfernung von Schwefelwasserstoff vorgeschlagen wird. Auch hier handelt es sich jedoch um eine mit K₂CO₃ imprägnierte Aktivkohle.

Auch in der Arbeit von K.-D. Henning und S. Schäfer "Impregnated activated carbon for environmental protection" (http://web.archive.org/web/20050408034843/http://www.activated-carbon.com/enviro.html) werden lediglich imprägnierte Aktivkohlen beschrieben.

Nachteil einer jeden Imprägnierung ist die damit verbundene Reduzierung der physikalischen Adsorptionskapazität der Ausgangskohlen. Durch das Imprägnierungsreagenz wird die Oberfläche teilweise belegt und dadurch das effektive Porenvolumen reduziert oder sogar blockiert. Imprägnierungsversuche an Formaktivkohlen auf Holzkohlebasis haben ergeben, dass die Iodzahl der Ausgangskohle von 1058 mg/g durch den Einbau von K₂CO₃ auf das Porensystem der Aktivkohle um 14 % abnimmt, wenn die Kohle 7.5 Gew.-% K₂CO₃ nach der Imprägnierung enthält. Die Adsorptionskapazität gemessen als CTC-Zahl verringert sich durch die K₂CO₃-Imprägnierung im Vergleich zur Ausgangskohle um etwa 10 Gew.-%. Daraus ist zu erkennen, dass die partielle Blockierung der Poren - insbesondere der Mikroporen - durch das Imprägnierungsreagenz verursacht wird. Ein Teil der Mikroporen bzw. Adsorptionsporen steht daher für den eigentlichen Adsorptionsprozess nicht mehr zur Verfügung. Es werden daher z. B. im Gasschutz sehr hoch aktivierte, feinporige Grundkohlen (spezifische Oberfläche: 1200 und 1500 m²g) als Ausgangsmaterialien für die Imprägnierung verwendet.

Von zentraler Bedeutung wäre es daher, den Zugang zu den katalytischen Zentren in den Mikroporen nicht zu beeinträchtigen, d. h. die Nachteile der Imprägnierung bezüglich Blockierung und/oder Verengung von Adsorptionsporen (Mikroporen) zu vermeiden, aber die Vorteile der Chemisorption bzw. der katalytisch wirkenden Substanzen in der Aktivkohle effektiv zu nutzen. In der älteren Patentanmeldung DE 10 2004 033 561 A1 wurde deswegen eine andere Strategie als die der nachträglichen Imprägnierung von Ausgangskohlen vorgeschlagen. In der Patentanmeldung wird die Herstellung einer sehr feinporigen Aktivkohle, d. h. mikroporösen Aktivkohle beschrieben, die neben der hohen Mikroporosität auch katalytisch aktive K₂CO₃-Zentren im Kohlenstoffgerüst aufweist, ohne dass diese die Blockierung/Verengmng von Poren - insbesondere von Mikroporen - bewirken. Der Ansatz zur Herstellung solcher Kohlen liegt in der speziellen K₂CO₃-Dotierung von Aktivkohlen, wobei der Dotierungsstoff schon im Vorwege in die Ausgangsstoffe (Kohlenstoffträger und/oder Bindemittel) vorzugsweise in den Kohlenstoffträger gegeben wird. Damit wird auch gewährleistet, dass bei der Herstellung der Aktivkohle (Karbonisierung, Wasserdampfaktivierung) das K₂CO₃ gleichmäßig im Kohlenstoffgerüst der hoch mikroporösen Aktivkohle letztendlich vorliegt.

Der Erfindung liegt die Aufgabe zu Grunde, ein Verfahren zur Eliminierung von Schwefelwasserstoff und Gerüchen aus Abwassersystemen zu schaffen, bei dem die Nachteile imprägnierter Aktivkohlen, d. h. die Reduzierung der physikalischen Adsorptionskapazität der Ausgangskohlen, vermieden werden.

Erfindungsgemäß wird dies durch die Verwendung von dotierten und katalytisch aktiven Formaktivkohlen als Abluftfilter erreicht.

In der vorliegenden Erfindung werden mikroporöse, K₂CO₃-dotierte Formaktivkohlen zur Entfernung von H₂S aus Gasströmen eingesetzt. Diese Formaktivkohlen enthalten katalytisch aktive K₂CO₃-Zentren im Kohlenstoffgerüst für die Reaktion mit Schwefelwasserstoff. Die Adsorptionsporen werden durch den Dotierungsstoff nicht verengt oder blockiert, insbesondere dann nicht, wenn der Dotierungsstoff gleichzeitig als katalytische Komponente für die Schwefelwasserstoff-Umsetzung und als Oxidationskatalysator für den Kohlenstoffträger zur Ausbildung des Porensystems genutzt wird, wie dies beim K₂CO₃ der Fall ist.

In einer weiteren vorteilhaften Ausgestaltung der Erfindung ist vorgesehen, dass K₂CO₃/MnO₂ dotierte Formaktivkohlen zur Entfernung von H₂S verwendet werden.

Die Beschreibung der Zugabe der oben genannten Dotierungsstoffe, die Bindemittelzugabe und die Herstellung der Formlinge, Karbonisat und Aktivat wird in der älteren Anmeldung DE 10 2004 033 561 A1 beschrieben und ist deswegen hier nicht weiter ausgeführt.

Eingesetzt und getestet - auch unter Praxisbedingungen - wurden mit K₂CO₃ dotierte Aktivkohlen zur katalytischen Entfernung von Schwefelwasserstoff als Abluftfilter für Abwassersysteme, z. B. in Pumpwerken zur Minderung von H₂S Emissionen unter der Geruchsschwelle von 0.15 mg/m³ (0.10 ppm) unter Umgebungsbedingungen (> 15 °C) ohne zusätzliche Hilfsmittel und/oder Energie.

Aktuelle Entwicklungen zur Herstellung von Aktivkohle mit erhöhter katalytischer Aktivität für saure Komponenten (SO₂, H₂S) gehen in eine andere Richtung und arbeiten mit der Einlagerung (somit Dotierung) von Kohlenstoffträgern mit stickstoffhaltigen Verbindungen. Aus den US Patenten Nr. 5,353,370; 5,356,849 und 5,444,031 sind z. B. Verfahren bekannt, die von einer Steinkohle ausgehen, die einer Niedertemperatur-Karbonisierung und Oxidation unterzogen wird, gefolgt von einem Kontaktieren mit einer geringen Menge einer stickstoffhaltigen Verbindung, wie z. B. Harnstoff. Die eigentliche Karbonisierung und Wasserdampfaktrvierung erfolgt erst nach dieser Vorbehandlung. Als Einsatzgebiet der so hergestellten katalytisch wirksamen Aktivkohlen wird die katalytische Umsetzung von H₂S, SOₓ und NOx angegeben. Die katalytische Aktivität ist dabei auf den Stickstoffanteil in der Aktivkohle zurückzuführen. Die Oberflächenchemie der Kohlenstoffmatrix durch Einführung von Heteroatomen, wie z.B. Stickstoff, spielt deswegen eine größe Rolle (vgl. die Publikation: A. Bagreev et al., Carbon 42 (2004) 469.

Das Patent WO 2004/052497 A2 offenbart die Herstellung von Filterelementen aus Aktivkohle mit Metalloxiden und einem Binder. Hierbei wird aber Aktivkohle mit dem Metalloxid gemischt ("blending of activated carbon") und mit einem Binder versehen. Die so erhaltene Masse wird anschließend geformt und kalziniert und so als Filterelement zur Entfernung von H₂S verwendet.

In der Veröffentlichung von "Z. Liu et al., Fuel 79 (2000) 1991" wird die Eisendotierung von Teer als Granulat mit Ferrocen mit anschließender Karbonisierung und Wasserdampfaktivierung beschrieben. Hier wurde der Einfluss von Eisen bzw. die entstehenden Eisenverbindungen (Fe, Fe₂O₃, Fe₃C) als Oxidationskatalysator für die Wasserdampfaktivierung beschrieben.

Das Verfahren nach der Erfindung weist erhebliche Vorteile auf. Die hierzu verwendete Formaktivkohle - mit spezifischen Oberflächen von mehr als 900 m²/g (BET-Oberfläche), mit Iodzahlen von größer als 1000 mg/g und mit einem Mikroporenvolumen von mehr als 0.38 ml/g - enthält die katalytisch aktive Komponente K₂CO₃ oder K₂CO₃ zusammen mit MnO₂ homogen in der porösen kohlenstoffhaltigen Matrix. Das Kaliumkarbonat erfüllt in dieser Weise zwei Funktionen: Einerseits dient es als Oxidationskatalysator zur Umsetzung des Kohlenstoffs bei der Aktivierung unter Bildung des Porensystems mit hohem Mikroporenanteil (> 0.38 ml/g), andererseits fungiert das K₂CO₃ als katalytische Komponente zur Entfernung von Schwefelwasserstoff aus Gasströmen. Es hat sich herausgestellt, dass die Zugabe von MnO₂ ebenso als aktive Komponente zur Entfernung von Schwefelwasserstoff wirkt. Dabei ist es unerheblich, ob das primär zugegebene MnO₂ während der weiteren Prozessschritte (Karbonisierung, Aktivierung) chemisch verändert wird (z. B. durch Reduktion zum MnO).

Vorteil des Dotierungsstoffes MnO₂ (oder die daraus entstehenden Produkte während der Herstellung) ist die praktische Wasserunlöslichkeit dieser Verbindungen (im Gegensatz zum K₂CO₃). Dies hat den Vorteil, dass die so dotierte Aktivkohle auch nach Fluten des Aktivkohlebettes mit Wasser seine katalytischen Eigenschaften gegenüber Schwefelwasserstoff weitgehend behält (Pufferfnnktion), da das Manganoxid nicht aus der porösen Aktivkohlestruktur herausgewaschen wird.

Es hat sich gezeigt, dass solch dotierte Aktivkohlen eine sehr hohe Adsorptionskapazität gegenüber Schwefelwasserstoff aufweisen. Dies wird darauf zurückgeführt, dass der Schwefelwasserstoff zunächst bevorzugt in den vorhandenen Mikroporen physikalisch adsorbiert und dadurch in den Mikroporen stark angereichert wird (Konzentrationseffekt). Anschließend wird das H₂S an der katalytisch aktiven Komponente (K₂CO₃ oder K₂CO₃/Manganoxid) vorwiegend in der Mikropore oxidiert (Sulfatbildung) bzw. zum Metallsulfid umgesetzt. Es ist auch charakteristisch für die in dem erfindungsgemäßen Verfahren eingesetzten H₂S-Eliminierungskatalysatoren, dass diese ihre katalytische Aktivität für H₂S behalten, auch wenn die Dotierungsstoffe extremen Temperaturbedingnngen (> 800 °C) unter den Herstellungsbedingungen (Karbonisierung, Aktivierung) in der Kohlenstoffmatrix ausgesetzt sind. Die leichte Erreichbarkeit des Katalysators in den Mikroporen durch H₂S ermöglicht eine starke KaLtalysator/Adsorbat Wechselwirkung. Es ist bekannt, dass durch die physikalische Adsorption in den Poren - insbesondere in den Mikroporen - Adsorptionswärme freigesetzt wird. Eine sehr starke Wärmetönung tritt insbesondere bei Kohlenstoffmolekularsieben auf, da hier der Porendurchmesser in etwa dem Moleküldurchmesser kleiner Moleküle entspricht (J.A. Allen et al. Carbon 37 (1999) 1485). Es wird daher davon ausgegangen, dass diese Wärmetönung, insbesondere in den Mikroporen der hier beschriebenen mikroporösen Formaktivkohlen, deutlich auftritt. Es ist bei mit K₂CO₃ imprägnierten Aktivkohlen bekannt, dass die katalytische Reaktion mit H₂S besondert gut bei ≥ 50 °C abläuft, K.D. Henning und S. Schäfer weisen auf deises Verhalten hin, vgl. Seite 10 http://www.activated-carbon.com/enviro.html. Das sehr gute Funktionieren dieser Formaktivkohlen auch bei z. B. < 15 °C weist darauf hin, dass eine lokale Wärmetönung bei den sehr mikroporösen und dotierten Kohlen auftritt. Diese in den Mikroporen auftretende lokale Wärmetönung würde die Weiterreaktion von H₂S mit K₂CO₃ unter Sulfatbildung auch bei niedrigen Umgebungstemperaturen wegen der lokalen Temperaturerhöhung in den Mikroporen beschleunigen (lokaler Temperatureffekt). Diese Tatsache ist ebenfalls ein Vorteil der hier eingesetzten mikroporösen und mit K₂CO₃ dotierten Aktivkohlen.

Des Wehren bleiben die Katalysatoren lange Zeit aktiv und die dotierte Formaktivkohle weist eine gute mechanische Stabilität und einen sehr geringen Druckverlust unter den in der Praxis gegebenen Strömungsgeschwindigkeiten in Abwasserbauwerken, z. B. Pumpwerken, auf. Die dotierte Aktivkohle kann leicht in großen Mengen hergestellt werden.

Eine Restfeuchte in der Aktivkohle ist für den Adsorptionsprozess von Vorteil, da hier eine Dissoziation von H₂S zu SH⁻/S²⁻ Ionen, insbesondere in den Mikroporen, begünstigt wird. Dies ist für die Reaktion mit Manganoxid wegen der Sulfid-Bildung von Vorteil. Im Gegensatz zur katalytischen Oxidation von H₂S an K₂CO₃ ist für die Reaktion von H₂S mit Manganoxid zum Mangansulfid kein Sauerstoff notwendig. Diese Reaktion findet deswegen auch unter anaeroben Reaktionsbedingungen statt.

Die Erfindung wird nachfolgend anhand von Beispielen näher erläutert:
a) Beladungsversuche mit Schwefelwasserstoff
Für die Beladungsversuche mit Schwefelwasserstoff an dotierter Formaktivkohle (Pelletdurchmesser: 4 mm) wurde eine Schüttung aus Aktivkohle mit folgenden Abmessungen verwendet: Schütthöhe: 5 cm, Durchmesser der Schüttung: 7.8 cm. Die Anströmfläche betrug daher 47.76 cm² und das Volumen der Schüttung 238.80 cm³. Es wurde ein Gasstrom von 0.06 m³/h (60 Liter/h) mit 16.7 Vol.-% H₂S-Gas in Luft zugeführt. Das H₂S-Gas wurde aus Eisensulfid (FeS) durch Zugabe von wässriger HCl-Lösung hergestellt. Somit war das Schwefelwasserstoffgas mit Wasserdampf gesättigt. Mit den angegebenen Daten ergibt sich eine Leerrohr-Strömungsgeschwindigkeit von 0.35 cm/s und eine mittlere Verweilzeit von 4.3 s bei einer Porosität der Schüttung (relativ freies Lückenvolumen) von ε = 0.30. Die Aktivkohle wurde in den Adsorber lose eingefüllt und die Oberfläche dann glattgestrichen. Die Gaszufuhr über das Aktivkohlebett wurde solange fortgesetzt, bis der H₂S-Detektor am Ausgang des Aktivkohlebettes 10 ppm H₂S anzeigte. Während der Beladung erwärmte sich die Aktivkohleschüttung. Bei Erreichen einer Temperatur von 50 °C wurde der Beladungsversuch unterbrochen, die Aktivkohleschüttung auf Raumtemperatur abgekühlt und erst dann der Beladungsversuch mit Schwefelwasserstoff fortgesetzt. Die Schwefelwasserstoffkapazität wurde über die Dauer der Gaszufuhr - bezogen auf 20 °C und Atmosphärendruck - und damit über die zugeführte H₂S-Menge bis zu dem Durchbruch von 10 ppm H₂S bestimmt.
Die Tabelle zeigt die Messergebnisse der Beladungsversuche. Es ist zu erkennen, dass die H₂S-Kapazität mit steigender K₂CO₃-Dotierung ansteigt. Bei einer Dotierung von 8.7 Gew.-% K₂CO₃ beträgt die H₂S-Kapazität 10.3 g/100 g Aktivkohle bei einer Iodzahl von 1170 mg/g. Wird die Dotierung auf 20.8 Gew.-% K₂CO₃ erhöht, dann wird auch - bei annähernd gleicher Iodzahl von 1200 mg/g - die H₂S-Beladung auf 19.0 g/100 g Aktivkohle gesteigert. Dies zeigt deutlich den Einfluss von K₂CO₃ bei etwa gleicher Oberfläche (Iodzahl) auf die H₂S-Kapazität. Des Weiteren kann auch MnO₂ als praktisch wasserunlöslicher Dotiertrungsstoff zur H₂S-Abscheidung verwendet werden. MnO₂ (Dotierung 5.8 Gew.-% im Aktivat) wurde zusammen mit K₂CO₃ (4.4 Gew.-% im Aktiva) als Dotierungsreagenz eingesetzt. Der H₂S-Beladungsversuch zeigt, dass die H₂S-Kapazität 14.4 g/100 g Aktivkohle beträgt. Diese Beladung ist größer als die Kapazität von mit 5.8 Gew.-% K₂CO₃ dotierter Aktivkohle (ohne MnO₂), die eine deutlich geringere Kapazität von nur 4.7 g/100 g aufweist. Somit kann MnO₂ auch als Dotierungsreagenz zur H₂S-Adsorption eingesetzt werden, selbst wenn MnO₂ während des Herstellungsprozesses (Karbonisierung, Aktivierung) möglicherweise chemisch verändert wird (z. B. zum MnO reduziert wird, das auch praktisch wasserunlöslich ist). Die mit K₂CO₃ und MnO₂ dotierte Kohle ist mit Wasser zur Entfernung des wasserlöslichen K₂CO₃ gewaschen worden. Die wasserbehandeite Probe zeigte dennoch eine H₂S-Kapazität von 10.7 g/100 g Aktivkohle auf und ist daher für die H₂S-Entfernung sehr gut geeignet.

**Tabelle: Beladungsversuche mit H₂S an dotierter Formaktivkohle**

| **Dotierungsstoff** | **Anteil Im Aktivat** | **experimentell gemessene** **H₂S-Kapazität** | **Iodzahl** **v.d. Bel.** | **Rütteldichte** **v.d. Bel.** | **Feuchte** **v.d. Bel.** |
|---|---|---|---|---|---|
| | Gew.-% | g H₂S / 100 g Aktivat | mg/g | g/l | Gew.-% |
| K₂CO₃ | 5.8 | 4.7 | 1077 | 482 | 3.5 |
| K₂CO₃ | 8.7 | 10.3 | 1170 | 451 | - 0 |
| K₂CO₃ | 20.8 | 19.0 | 1200 | 388 | - 0 |
| K₂CO₃ + MnO₂ | 4.4 + 5.8^{*} | 14.4 (10.7)^{†} | 1240 | 428 | 1.76 |

| | | | | | |
|---|---|---|---|---|---|
| Anmerkungen: v.d. Bel.: vor der H₂S-Beladung; ^{#}evtl. chemische Veränderungen von MnO₂ während des Herstellungsprozesses ist nicht berücksichtigt worden; ^{†} Probe mit 4.4 Gew.-% K₂CO₃ und 5.8 Gew.-% MnO₂ mit Wasser (zur Entfernung von K₂CO₃) gewaschen und H₂S-Kapazität bestimmt | | | | | |

b) Der Einsatz der Filter unter Praxisbedingungen wird anhand von Abbildungen näher erläutert.
Es zeigen:
- Fig. 1: die Schwefelwasserstoffkonzentration in einem Abwasserpumpwerk und die Schwefelwasserstoffkonzentration nach Einbau des Filters nach dem Filter;
- Fig. 2: die Schwefelwasserstoffkonzentration im Pumpwerk mit und ohne Filter bei niedrigen Temperaturen;
- Fig. 3: die Schwefelwasserstoffkonzentration im Pumpwerk mit und ohne Filter bei mittleren Temperaturen;
- Fig. 4: den Druckverlust über unterschiedliche Aktivkohleschüttungen als Funktion der Leerrohrgeschwindigkeit.

Eine mit 5.8 Gew.-% K₂CO₃ dotierte Formaktivkohle (Pelletdurchmesser 4 mm, Rütteldichte 482 g/l) wurde für Versuche im Abwasserpumpwerk zur Reduzierung der Schwerfelwasserstoffkonzentration im Gasstrom (Abwasserabluft) eingesetzt. Die spezifische Oberfläche (BET-Oberfläche nach DIN 66131) der dotierten Kohle betrug 950 m²/g und die Iodzahl 1077 mg/g. Das Mikroporenvolumen wurde aus der Stickstoffadsorption bei 77 K nach einer Auswertung von Horvath and Kawazoe (DIN 66 135, Teil 4) bestimmt und beträgt 0.41 ml/g.

Es wurde eine Aktivkohleschüttung mit den Abmessungen (Schütthöhe: 4.6 cm, Fläche: 65.5 cm × 35,5 cm) eingesetzt. Der Volumenstrom des zugeführten schwefelwasserstoffhaltigen Gasstromes (Abwasserabluft) betrug im Mittel 400 Liter/h (Volumenstrom eines Pumpenspiels in der Zeit zwischen den Pumpvorgängen). Daraus errechnet sich im Aktivkohlefilter eine Strömungsgeschwindigkeit von w **=** 0.048 cm/s und eine mittlere Verweilzeit von τ = 28.8 s bei einer Porosität der Schüttung von ε = 0.30. Diese Verweilzeit reicht bei weitem aus, um Chemisorptionsprozesse (hier Umsetzung von Schwefelwasserstoff am K₂CO₃) vollständig ablaufen zu lassen, da Chemisorptionsprozesse zwar grundsätzlich - im Vergleich zur physikalischen Adsorption - langsamer ablaufen, aber trotzdem innerhalb von etwa 10 s abgeschlossen sind.

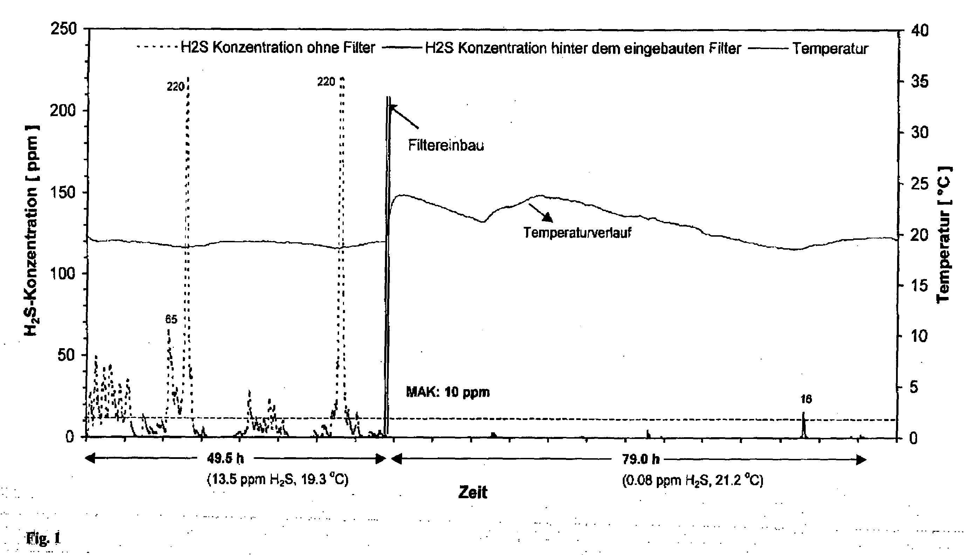

In Fig. 1 ist die Schwefelwasserstoffkonzentration in ppm in einem Abwasserpumpwerk dargestellt. Die durchschnittliche Schwefelwasscrstoffkonzentration im Pumpwerk (ohne Filter) beträgt 13,5 ppm über einen Zeitraum von 49.5 h mit Konzentrationsspitzen bis etwa 220 ppm bei einer mittleren Temperatur von 19.3 °C. Nach Einbau des Aktivkohlefilters wurde die Schwefelwasserstoffkonzentration deutlich reduziert. Die durchschnittliche H₂S-Konzentration betrug dann nur noch 0.08 ppm (gemessen nach dem Filter) über einen Zeitraum von 79 Stunden mit Spitzen bis maximal 1.6 ppm bei einer durchschnittlichen Temperatur von 21.2 °C.

Die Messung der Konzentration erfolgte mittels eines Gasprüfgerätes, ohne dass die Umgebungsbedingungen innerhalb des Pumpwerkes verändert wurden. Aus der Darstellung wird deutlich, dass die teilweise gesundheitsschädlichen Konzentrationen von H₂S durch den Einsatz der dotierten, mikroporösen Aktivkohle mit 5.8 Gew.-% K₂CO₃ bei einer mittleren Verweilzeit von etwa 29 s eliminiert werden können, da die mittlere Schwefelwasserstoffkonzentration von 0.08 ppm unterhalb der Geruchsschwelle von 0.10 ppm (oder 0. 15 mg/m³) liegt und Konzentrationsspitzen fast gänzlich reduziert und damit abgepuffert werden.

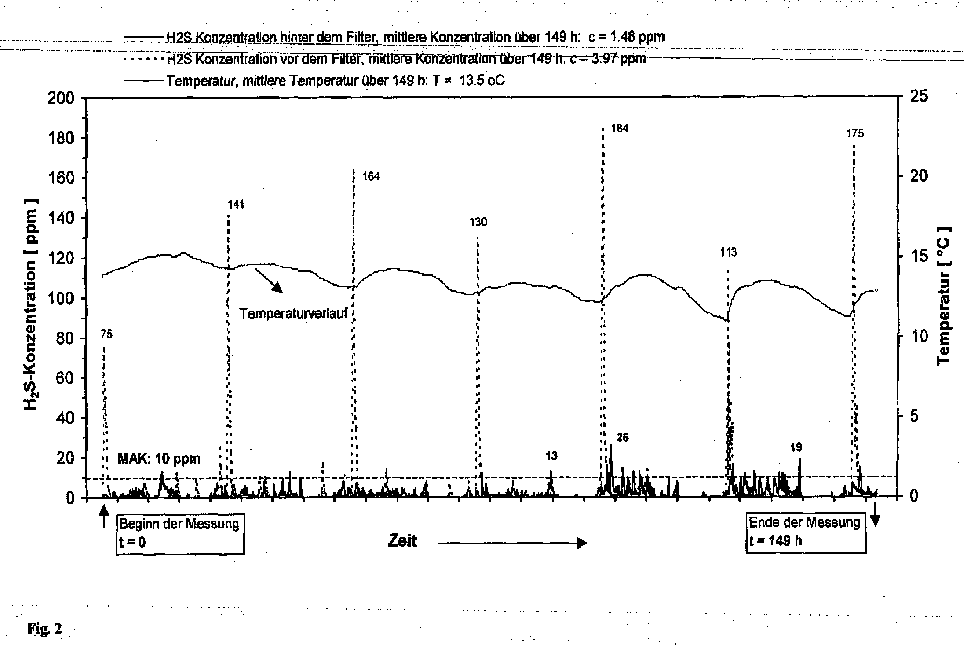

Fig. 2 zeigt die Schwefelwasserstoffkonzentration im Pumpwerk mit und ohne Filtersystem bei relativ niedrigen Temperaturen. Der Filter war bis dahin ein halbes Jahr in Betrieb und der Wassergehalt (Feuchte) der Aktivkohle war sehr hoch und betrug etwa 35 Gew.-%. Die durchschnittliche Schwefelwasserstoffkonzentration im Pumpwerk betrug 3.97 ppm über einen Zeitraum von 149 Stunden mit Konzentrationsspitzen bei 75, 141, 164, 13 0, 184, 113 und 175 ppm bei einer mittleren Temperatur von nur 13.5 °C. Nach dem Filter wurde eine mittlere Schwefelwasserstoffkonzentration von 1.48 ppm über einen Zeitraum von 149 Stunden gemessen mit Konzentrationsspitzen bei 13, 26 und 19 ppm bei einer mittleren Temperatur von nur 13,5 °C. Dies zeigt deutlich, dass selbst bei relativ niedrigen Temperaturen (mittlere Temperatur 13.5 °C) und bei hoher Feuchtigkeit (ca. 35 Gew.-% Wasser) in der Formaktivkohle die Konzentrationsspitzen an Schwefelwasserstoff durch den Aktivkohlefilter abgepuffert werden.

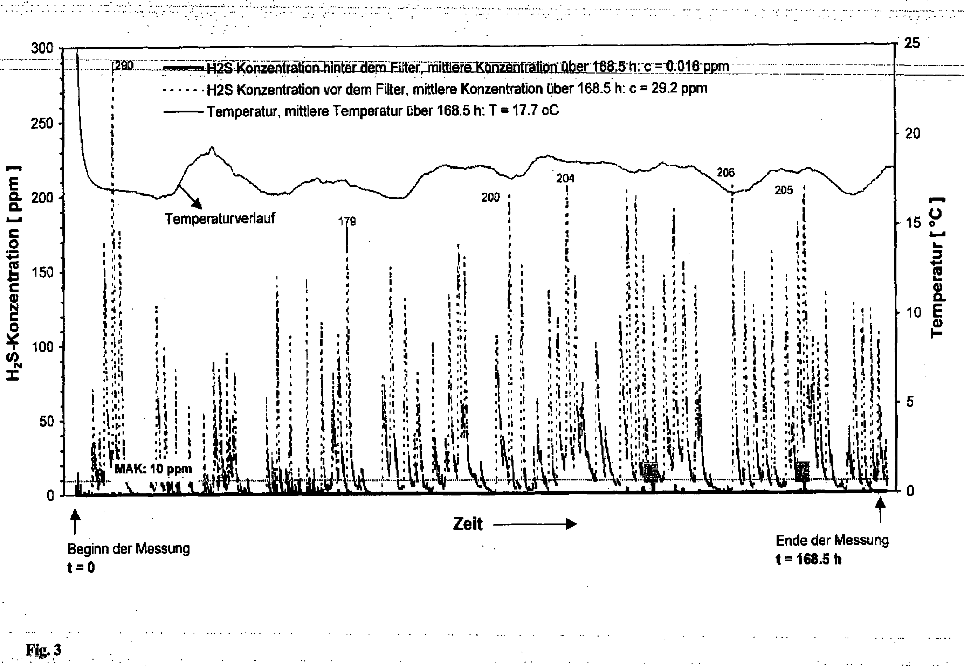

Fig. 3 zeigt die Schwefelwasserstoffkonzentration in ppm in einem Abwasserpumpwerk mit und ohne Filtersystem bei einer mittleren Temperatur von 17.7 °C. Der Filter war bis dahin ein Jahr in Betrieb. Die durchschnittliche Schwefelwasserstoffkonzentration im Pumpwerk betrug 29.2 ppm über einen Zeitraum von 168.5 Stunden mit vielen Konzentrationsspitzen um 200 ppm. Damit lag die durchschnittliche Schwefelwasserstoffkonzentration über dem MAK-Wert von 10 ppm. Nach dem Filter wurde eine mittlere Sehwefelwasserstoffkonzentration von nur 0.0126 ppm über einen Zeitraum von 168.5 Stunden gemessen. Die Konzentrationsspitzen lagen unterhalb von 10 ppm. Damit lag die durchschnittliche Schwefelwasserstoffkonzentration nach dem Filter unterhalb der Geruchsschwelle von 0.1 ppm. Die Konzentrationsspitzen wurden praktisch vollständig abgepuffert.

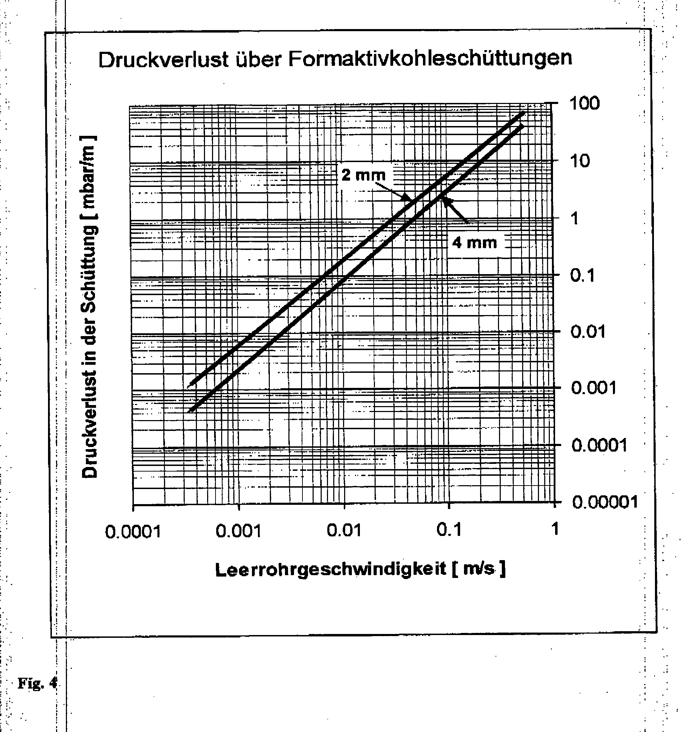

Aufgrund der speziellen Form der hochwertigen dotierten Formaktivkohle ist der Druckverlust in der Aktivkohleschüttung sehr gering. Fig. 4 zeigt den Druckverlust in mbar/m über Aktivkohleschüttungen von Formaktivkohle verschiedener Durchmesser (2 und 4 mm) als Funktion der Leerrohrgeschwindigkeit in m/s. Bei der berechneten Leerrohrgeschwindigkeit von 0.0005 m/s (s.o.) beträgt der Druckverlust in der Schüttung (Schütthöhe: 4.6 cm) nur 0.037 µbar und ist daher praktisch zu vernachlässigen.

## Patentansprüche

1. Verfahren zur Eliminierung von Schwefelwasserstoff und Gerüchen in Abwassersystemen durch die Verwendung von dotierten und katalytisch aktiven Formaktivkohlen als Abluftfilter, **dadurch gekennzeichnet, dass** die eingesetzte Formaktivkohle mit der Komponente K₂CO₃ oder mit der Komponenten K₂CO₃/MnO₂ dotiert wird.

2. Verfahren nach Anspruch 1, **dadurch gekennzeichnet, dass** die eingesetzte mikroporöse Formaktivkohle die Komponente MnO₂ als wasserunlösliche Komponente enthält.

## Claims

1. A process for eliminating hydrogen sulfide and odors in wastewater systems by using doped and catalytically active molded activated carbons as exhaust air filters, **characterized in that** the molded activated carbon used is doped with the component K₂CO₃ or with the component K₂CO₃/MnO₂.

2. The process according to claim 1, **characterized in that** the microporous molded activated carbon used comprises the component MnO₂ as water-insoluble component.

## Revendications

1. Procédé destiné à éliminer le sulfure d'hydrogène et les odeurs présents dans des réseaux d'eau usées en utilisant, en tant que filtre de sortie d'air, différents charbons actifs agglomérés dotés et présentant une activité catalytique, **caractérisé en ce que** le charbon actif aggloméré mis en oeuvre est doté par le composant K₂CO₃ ou par le composant K₂CO₃/MnO₂.

2. Procédé selon la revendication 1, **caractérisé en ce que** le charbon actif aggloméré microporeux mis en oeuvre contient le composant MnO₂ en tant que composant insoluble dans l'eau.
